# EUROPEAN PATENT SPECIFICATION

(11) **EP 2 620 138 B1**
(45) Date of publication and mention of the grant of the patent: **07.12.2016**
(21) Application number: 11798298.3
(22) Date of filing: 22.09.2011
(51) Int. Cl.: A61K 8/73, A61K 8/02, A61K 8/19, A61K 8/34, A61K 8/365, A61K 9/46, A61K 47/12, A61K 47/38, A61Q 19/00, A61K 8/49, A61K 9/00, A61K 9/12, A61K 8/04

(54) **FOAM-TYPE EXTERNAL SKIN PREPARATION**
SCHAUMARTIGES EXTERNES HAUTPRÄPARAT
PRÉPARATION CUTANÉE EXTERNE DE TYPE MOUSSE

(30) Priority: 24.09.2010 JP 2010213344
(43) Date of publication of application: 31.07.2013
(73) Proprietor: Daido Chemical Corporation, Osaka-shi Osaka 555-0011 (JP)
(72) Inventor: INAMOTO, Yasunari, Osaka-shi Osaka 555-0011 (JP); SHIMAMOTO, Toshio, Osaka-shi Osaka 555-0011 (JP)
(74) Representative: Nieuwenhuys, William Francis
(86) International application number: PCT/JP2011/071734
(87) International publication number: WO 2011/162421

(56) References cited:
- EP-A1- 1 386 613
- WO-A1-02/080941
- JP-A- H09 110 901
- JP-A- S55 110 103
- JP-A- 2008 524 227
- US-A- 4 228 277
- US-A1- 2006 134 047
- TAKAYUKI KITAMURA ET AL.: 'Tokushu Koshohin-yo Zonen Gel-kazai no Kaihatsu to Oyo, Characteristic of hydrophobically-modified hydroxypropyl methylcellulose, and application of hair cosmetics' FRAGRANCE JOURNAL vol. 35, no. 7, 15 July 2007, pages 72 - 77, XP008169152

## Description

### TECHNICAL FIELD

The present invention relates to a foam-type external skin preparation generating carbon dioxide, in which the foaming properties and carbon dioxide-holding properties are excellent.

### BACKGROUND ART

Carbon dioxide has been known to have vasodilation. In clinical applications, carbon dioxide has been used for rehabilitation such as a carbon dioxide bath. A pack preparation using carbon dioxide generated by mixing a carbonate and an acid has been conventionally known as a skin care preparation (Patent Document 1).

However, carbon dioxide is released in the air immediately after generation. Therefore, in use of carbon dioxide for such a pack preparation, there has been a problem of how to continuously bring the carbon dioxide into contact with the skin.

As a method for solving the problem, there is a method in which a thickening composition containing a carbonate and a water-soluble thickener such as an alginate or sodium carboxymethylcellulose, and a water-soluble acid are mixed immediately prior to use, to generate carbon dioxide in a base material having a viscosity beforehand, and as a result the released amount of carbon dioxide is decreased (Patent Document 2).

When importance is attached to usability in which a preparation does not sag from the skin due to an appropriate tackiness in use to prevent clothes from getting dirty, the composition is in a paste form. In this case, foaming of carbon dioxide during mixing of the composition with the water-soluble acid is sufficiently mild, and it is difficult that the generation of carbon dioxide appeals to the eye of the user. When the kinds of carbonate and water-soluble acid are changed to increase the amount of carbon dioxide foam, the generation of carbon dioxide can appeal to the eye of the user. However, the viscosity of the composition is remarkably decreased by generated gas, and the preparation may sag from the skin in use to get clothes dirty.

Therefore, it is difficult to obtain a foam-type external skin preparation exhibiting an amount and retention of carbon dioxide foam appealing to the eye of the user, as well as good usability such as prevention of the preparation sagging from the skin.

### PRIOR ART DOCUMENTS

### PATENT DOCUMENTS

Patent Document 1: JP-A-60-215606
Patent Document 2: Japanese Patent No. 4248878
Patent Document 3: JP-A-2004-238333

### DISCLOSURE OF THE INVENTION

### PROBLEMS TO BE SOLVED BY THE INVENTION

An object of the present invention is to provide a foam-type external skin preparation exhibiting an amount of and a retention of carbon dioxide foam appealing to the eye of the user, as well as good usability, such as in preventing the preparation from sagging from the skin, neither of which can be easily obtained with conventional foam-type external skin preparations.

### SOLUTIONS TO THE PROBLEMS

The inventors have intensively studied the problems, and found a technique providing a foam-type external skin preparation which has an excellent retention of carbon dioxide foam by using a hydrophobically modified alkyl cellulose, and improved sense of use such as prevention of the preparation sagging from the skin. Thus, the present invention has been completed.

The present invention provides the following foam-type external skin preparations.
Item 1. A foam-type external skin preparation comprising a hydrophobically modified alkyl cellulose; at least one salt selected from the group consisting of a carbonate and a bicarbonate; and a water-soluble acid, wherein the hydrophobically modified alkyl cellulose is represented by the following structural formula (1): 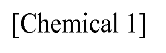 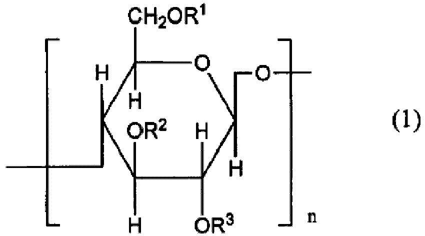 wherein R¹, R², and R³ are the same or different, and are a hydrogen atom, a lower alkyl group, a -[CH₂CH₂-ₖ(CH₃)ₖO]ₘH group, or a -CH₂CH(OH)CH₂OCⱼH₂ⱼ₊₁ group; n is an integer of 100 to 10,000; k is an integer of 0 or 1; m is an integer of 1 to 10; and j is an integer of 6 to 26, and the hydrophobically modified alkyl cellulose necessarily contains a -CH₂CH(OH)CH₂OCⱼH₂ⱼ₊₁ group;
   wherein the lower alkyl group is a methyl group or an ethyl group.
Item 2. The foam-type external skin preparation according to item 1, wherein the hydrophobically modified alkyl cellulose represented by the structural formula (1) contains 10.0 to 50.0% by mass of lower alkyl group, 3.0 to 20.0% by mass of -[CH₂CH₂₋ₖ(CH₃)ₖO]ₘH group, and 0.1 to 10.0% by mass of -CH₂CH(OH)CH₂OCⱼH₂ⱼ₊₁ group.
Item 3. The foam-type external skin preparation according to item 1 or 2, wherein the value j of the -CH₂CH(OH)CH₂OCⱼH₂ⱼ₊₁ group in the structural formula (1) is 18.
Item 4. The foam-type external skin preparation according to any one of items 1 to 3, wherein the at least one salt selected from the group consisting of a carbonate and a bicarbonate is sodium bicarbonate.
Item 5. The foam-type external skin preparation according to any one of items 1 to 4, wherein the water-soluble acid is a hydroxy acid.
Item 6. The foam-type external skin preparation according item 5, wherein the hydroxy acid is citric acid.
Item 7. The foam-type external skin preparation according to any one of items 1 to 6, further containing a polyhydric alcohol.
Item 8. The foam-type external skin preparation according to item 7, wherein the polyhydric alcohol contains one or more members selected from the group consisting of propylene glycol, dipropylene glycol, 1,3-butylene glycol, and 1,2-pentanediol.
Item 9. The foam-type external skin preparation according to any one of items 1 to 8, wherein the foam-type external skin preparation comprises a preparation A containing at least one salt selected from the group consisting of a carbonate and a bicarbonate and a preparation B containing a water-soluble acid, the preparation A and preparation B having been kept apart and mixed before use.
Item 10. A method for preparing a foam-type external skin preparation, wherein the foam-type external skin preparation comprises: a hydrophobically modified alkyl cellulose; at least one salt selected from the group consisting of a carbonate and a bicarbonate; and a water-soluble acid; wherein the hydrophobically modified alkyl cellulose is represented by the following structural formula (1): 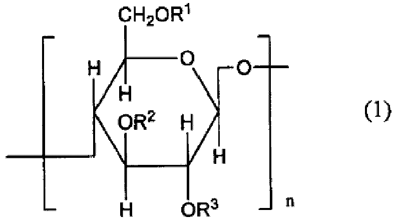 wherein R¹, R² and R³ are the same or different, and are a hydrogen atom, a lower alkyl group a -[CH₂CH₂₋ₖ(CH₃)ₖO]ₘH group, or a -CH₂CH(OH)CH₂OCⱼH₂ⱼ₊₁ group; n is an integer of 100 to 10,000; k is an integer of 0 or 1; m is an integer of 1 to 10; and j is an integer of 6 to 26; and the hydrophobically modified alkyl cellulose necessarily contains a -CH₂CH(OH)CH₂OCⱼH₂ⱼ₊₁ group, wherein the lower alkyl group is a methyl group or an ethyl group;
   the method comprising:
   mixing before use a preparation A containing at least one salt selected from the group consisting of a carbonate and bicarbonate and a preparation B containing a water-soluble acid, the preparation A and the preparation B having been kept apart.
Item 11. A method of use of a foam-type external skin preparation, wherein the foam type external preparation comprises: a hydrophobically modified alkyl cellulose; at least one salt selected from the group consisting of a carbonate and bicarbonate; and a water-soluble acid, wherein the hydrophobically modified alkyl cellulose is represented by the following structural formula (1): 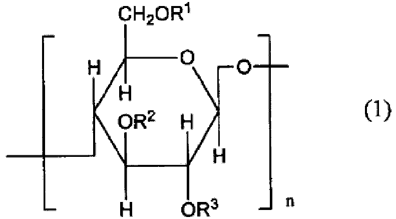 wherein R¹, R² and R³ are the same or different, and are a hydrogen atom, a lower alkyl group a -[CH₂CH₂₋ₖ(CH₃)ₖO]ₘH group, or a -CH₂CH(OH)CH₂OCⱼH₂ⱼ₊₁ group; n is an integer of 100 to 10,000; k is an integer of 0 or 1; m is an integer of 1 to 10; and j is an integer of 6 to 26; and the hydrophobically modified alkyl cellulose necessarily contains a -CH₂CH(OH)CH₂OCⱼH₂ⱼ₊₁ group, wherein the lower alkyl group is a methyl group or an ethyl group, and wherein the foam-type external skin preparation comprises a preparation A containing at least one salt selected from the group consisting of a carbonate and a bicarbonate and a preparation B containing a water-soluble acid, the preparation A and the preparation B having been kept apart and mixed before use.
Item 12. The foam-type external skin preparation according to any one of items 1-9, wherein the foam-type external skin preparation is a pack cosmetic material used for the face.

### EFFECTS OF THE INVENTION

A foam-type external skin preparation contains a hydrophobically modified alkyl cellulose as an essential component, and therefore can exhibit an amount and a retention of carbon dioxide foam and good usability such as prevention of the preparation sagging from the skin.

### DETAILED DESCRIPTION OF PREFERRED EMBODIMENTS

The foam-type external skin preparation of the present invention contains a hydrophobically modified alkyl cellulose as an essential component. The foam-type external skin preparation has an excellent retention of carbon dioxide foam by using a hydrophobically modified alkyl cellulose, and improved sense of use such as prevention of the preparation sagging from the skin.

The foam-type external skin preparation of the present invention comprises a hydrophobically modified alkyl cellulose; at least one salt selected from the group consisting of a carbonate and a bicarbonate; and a water-soluble acid.

Hereinafter, the hydrophobically modified alkyl cellulose; the at least one salt selected from the group consisting of a carbonate and a bicarbonate; and the water-soluble acid will be described.

### (1) Hydrophobically modified alkyl cellulose

A hydrophobically modified alkyl cellulose used for the foam-type external skin preparation of the present invention is obtained by introducing a long-chain alkyl group that is a hydrophobic group into a cellulose ether derivative.

The hydrophobically modified alkyl cellulose is represented by the following structural formula (1): 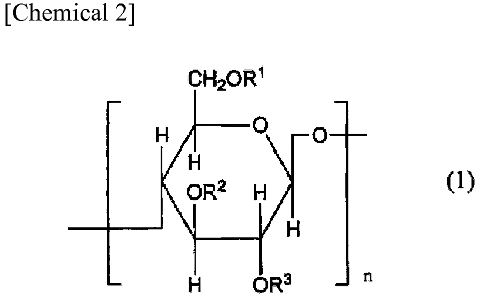 wherein R¹, R², and R³ are the same or different, and are a hydrogen atom, a lower alkyl group, a -[CH₂CH₂₋ₖ(CH₃)ₖO]ₘH group, or a -CH₂CH(OH)CH₂OCⱼH₂ⱼ₊₁ group; n is an integer of 100 to 10,000; k is an integer of 0 or 1; m is an integer of 1 to 10; and j is an integer of 6 to 26, and the hydrophobically modified alkyl cellulose necessarily contain a -CH₂CH(OH)CH₂OCⱼH₂ⱼ₊₁ group; wherein the lower alkyl group is a methyl group or an ethyl group.

Among cellulose ether derivatives which are a base of a hydrophobically modified alkyl cellulose, hydroxypropyl methyl cellulose is suitably selected from the viewpoint of availability, etc. Hydroxypropyl methyl cellulose (HPMC) is represented by the structural formula (1), wherein R¹, R², and R³ are the same or different, and are a hydrogen atom (-H), a methyl group (-CH₃), or a -[CH₂CH(CH₃)O]ₘH group, and HPMC contains a -[CH₂CH(CH₃)O]ₘH group.

A hydrophobically modified alkyl cellulose is obtained by introducing a long-chain alkyl group into HPMC. For example, a hydrophobically modified alkyl cellulose can be produced by reacting HPMC with the stearyl glycidyl ether represented by the formula below in the presence of alkaline catalyst.

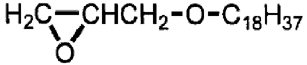

In addition to stearyl glycidyl ether, cetyl glycidyl ether or decyl glycidyl ether may be used.

It is suitable that CⱼH₂ⱼ₊₁ in a -CH₂CH(OH)CH₂OCⱼH₂ⱼ₊₁ group that is a hydrophobic group introduced into a hydrophobically modified alkyl cellulose be a stearyl group (-C₁₈H₃₇) since retention of foam is imparted, sagging from the skin is prevented, and the sense of use is good As a result, the -CH₂CH(OH)CH₂OCⱼH₂ⱼ₊₁ group is converted into a -CH₂CH(OH)CH₂O-C₁₈H₃₇ group.

The hydrophobically modified alkyl cellulose represented by the structural formula (1) preferably contains a -CH₂CH(OH)CH₂OCⱼH₂ⱼ₊₁ group in a content of 0.1 to 10.0% by mass, more preferably 0.1 to 2.0% by mass, and particularly preferably 0.1 to 1.0% by mass. When the content of CⱼH₂ⱼ₊₁ in the -CH₂CH(OH)CH₂OCⱼH₂ⱼ₊₁ group is more than 10.0% by mass in the hydrophobically modified alkyl cellulose, the solubility to water is low and a dissolved solution tends to become clouded. Therefore, an end product is not likely to obtain sufficient viscosity effective in retention of foam.

The lower alkyl group in the hydrophobically modified alkyl cellulose represented by the structural formula (1) is a methyl group or an ethyl group, preferably a methyl group.

Since the hydrophobically modified alkyl cellulose represented by the structural formula (1) has a good solubility to water, the content of a methyl group is preferably 10.0 to 50.0% by mass, and more preferably 21.5 to 30.0% by mass in the hydrophobically modified alkyl cellulose.

The -[CH₂CH₂-ₖ(CH₃)ₖO]ₘH group in the hydrophobically modified alkyl cellulose represented by the structural formula (1) is a -[CH₂CH₂O]ₘH group when k is 0, and a -[CH₂CH(CH₃)O]ₘH when k is 1. A -[CH₂CH(CH₃)O]ₘH group when k is 1 is particularly preferable.

From the viewpoint of solubility to water, the hydrophobically modified alkyl cellulose represented by the structural formula (1) preferably contains a -[CH₂CH(CH₃)O]ₘH group in an amount of 3.0 to 20.0% by mass, and more preferably 7.0 to 11.0% by mass in the hydrophobically modified alkyl cellulose.

The contents of the lower alkyl group, -[CH₂CH₂-ₖ(CH₃)ₖO]ₘH group, and -CH₂CH(OH)CH₂OCⱼH₂ⱼ₊₁ group in the hydrophobically modified alkyl cellulose are values measured by a method in accordance with The Japanese Pharmacopoeia 13th edition, the section hydroxypropyl methyl cellulose 2208.

One example of a hydrophobically modified alkyl cellulose satisfying these conditions is preferably SANGELOSE available from Daido Chemical Corporation.

The amount of mixed hydrophobically modified alkyl cellulose used for the foam-type external skin preparation of the present invention is preferably 0.1 to 10.0% by mass, and more preferably 0.5 to 5.0% by mass based on the whole amount of the foam-type external skin preparation of the present invention. When the amount of mixed hydrophobically modified alkyl cellulose based on the whole amount of the foam-type external skin preparation is less than 0.1% by mass, an end product tends to reduce sufficient thickening effect and an effect of holding carbon dioxide gas (foam). When it is more than 10.0% by mass, the viscosity is too high, and mixing is difficult in use. Further, the end product is difficult to apply to the skin. Moreover, this tends to pose a problem for production of the product.

Hereinafter, components which are other than a hydrophobically modified alkyl cellulose and preferably contained in the foam-type external skin preparation of the present invention will be described. Each component is represented by mixing ratio (% by mass) based on the whole amount (or each form) of the foam-type external skin preparation and mixed amount (part by mass) based on 100 parts by mass of hydrophobically modified alkyl cellulose.

### (2) At least one salt selected from the group consisting of carbonate and bicarbonate

At least one salt selected from the group consisting of a carbonate and a bicarbonate used for the foam-type external skin preparation of the present invention (hereinafter sometimes referred to as "carbonates") can be used without particular restriction as long as it can be used for a cosmetic material. Examples thereof may include sodium carbonate, sodium bicarbonate, potassium carbonate, potassium bicarbonate, and calcium carbonate. Among them, sodium bicarbonate is particularly preferable in terms of foaming characteristics.

The mixing amount of at least one salt selected from the group consisting of a carbonate and a bicarbonate used for the foam-type external skin preparation of the present invention is preferably 0.1 to 10.0% by mass, more preferably 0.5 to 5.0% by mass, and further preferably 0.8 to 3.0% by mass, based on the whole amount of the foam-type external skin preparation. When the mixing amount of at least one salt selected from the group consisting of a carbonate and a bicarbonate based on the whole amount of the foam-type external skin preparation is less than 0.1% by mass, foaming due to carbon dioxide gas is unlikely to be sufficiently achieved. When it is more than 10.0% by mass, a large amount of carbon dioxide gas is generated. Therefore, the product may irritate the skin in use.

For the same reason, the amount of mixed carbonates in the foam-type external skin preparation (whole amount) of the present invention is preferably about 1.0 to 5,000 parts by mass, and more preferably about 10 to 1,000 parts by mass based on the 100 parts by mass of hydrophobically modified alkyl cellulose.

### (3) Water-soluble acid

A water-soluble acid has an action of generating carbon dioxide as a gas component for formation of foam by reaction with at least one salt selected from the group consisting of a carbonate and a bicarbonate in the use of the foam-type external skin preparation of the present invention.

As a water-soluble acid used for the foam-type external skin preparation of the present invention, a water-soluble acid which reacts with at least one salt selected from the group consisting of a carbonate and a bicarbonate to generate carbon dioxide gas can be used. Specifically, a water-soluble acid, such as malic acid, succinic acid, oxalic acid, maleic acid, citric acid, glycolic acid, mandelic acid, tartaric acid, and glucono delta lactone (which becomes gluconic acid in an aqueous solution), is preferably exemplified. A hydroxy acid such as citric acid, glycolic acid, mandelic acid, and tartaric acid, and glucono delta lactone (gluconic acid) are further preferable in terms of affinity during mixing in a final composition. Only one kind of water-soluble acid as described above may be contained, or a combination of two or more kinds may be contained.

The amount of mixed water-soluble acid used for the foam-type external skin preparation of the present invention is preferably 0.5 to 10.0% by mass, and more preferably 1.0 to 5.0% by mass based on the whole amount of the foam-type external skin preparation of the present invention. When the amount of mixed water-soluble acid based on the whole amount of the foam-type external skin preparation is less than 0.5% by mass or more than 10.0% by mass, the pH of the foam-type external skin preparation tends to be unable to be well adjusted, and therefore it tends to be difficult to generate carbon dioxide.

For the same reason, the amount of mixed water-soluble acid in the foam-type external skin preparation (whole amount) of the present invention is preferably about 5.0 to 5,000 parts by mass, and more preferably about 10 to 1,000 parts by mass based on the 100 parts by mass of hydrophobically modified alkyl cellulose.

### (4) Polyhydric alcohol

In terms of purpose for uniformity of physical properties of foam-type external skin preparation and stability of product, it is preferable that a polyhydric alcohol be further contained in the foam-type external skin preparation of the present invention.

Examples of polyhydric alcohol may include a polyhydric alcohol having 2 to 6 carbon atoms and 2 to 3 oxygen atoms. Specifically, a polyhydric alcohol, such as ethylene glycol, diethylene glycol, propylene glycol, 1,3-propanediol, dipropylene glycol, 1,3-butylene glycol, 1,2-pentanediol, 1,2-hexanediol, 3-methyl-1,3-butanediol, glycerin, and sorbitol is preferably exemplified. Among them, propylene glycol, dipropylene glycol, 1,3-butylene glycol, and 1,2-pentanediol are particularly preferable in terms of availability and addition of sense of use.

The amount of mixed polyhydric alcohol is preferably 0.1 to 50.0% by mass based on the whole amount of foam-type external skin preparation. A more preferable form is a form containing 10.0 to 30.0% by mass of polyhydric alcohol. When the amount of mixed polyhydric alcohol based on the whole amount of foam-type external skin preparation is less than 0.1% by mass, the physical properties of thickening composition are not uniform (lumps), and a mixing process in use and application to the skin are difficult. When it is more than 50.0% by mass, stickiness is increased in use. Therefore, the sense of use is not good.

For the same reason, the amount of mixed polyhydric alcohol in the foam-type external skin preparation (whole amount) of the present invention is preferably about 1.0 to 50,000 parts by mass, and more preferably about 200 to 6,000 parts by mass based on the 100 parts by mass of hydrophobically modified alkyl cellulose.

### (5) Water

The foam-type external skin preparation of the present invention has an action of generating carbon dioxide as a gas component for formation of foam by reaction of at least one salt selected from the group consisting of a carbonate and a bicarbonate with the water-soluble acid during use. The carbon dioxide gas is held in a gel including the hydrophobically modified alkyl cellulose and water and having a thickening effect.

The amount of mixed water used for the foam-type external skin preparation of the present invention is preferably 30 to 99.3% by mass, and more preferably 57 to 88.5% by mass based on the whole amount of the foam-type external skin preparation of the present invention. When the amount of mixed water based on the whole amount of the foam-type external skin preparation is less than 30% by mass, the viscosity is too high, and mixing tends to be difficult during use. Further, application to the skin tends to be difficult. Moreover, this tends to pose a problem for production of the product. When it is more than 99.3% by mass, the end product tends to reduce sufficient thickening effect and an effect of holding carbon dioxide gas (foam).

For the same reason, the amount of mixed water in the foam-type external skin preparation (whole amount) of the present invention is preferably 300 to 99,300 parts by mass, and more preferably 1,140 to 17,700 parts by mass based on the 100 parts by mass of hydrophobically modified alkyl cellulose.

### (6) Other component

The foam-type external skin preparation of the present invention can contain any component which is generally used for a cosmetic material in addition to the essential components. Examples of any component may include hydrocarbon such as squalane, liquid paraffin, and vaseline, liquid oils and fats such as olive oil, macadamia nut oil, and castor oil, solid oils and fats such as coconut oil, palm oil, and shea oil, wax such as beeswax, carnauba wax, and lanolin, higher alcohols such as stearyl alcohol, behenyl alcohol, and isostearyl alcohol, synthetic ester oil such as cetyl octanoate, isopropyl palmitate, and glyceryl tri-2-ethylhexanoate, silicone oil such as dimethyl polysiloxane, decamethyl cyclopentasiloxane, a silicone resin, and amino-modified polysiloxane, a lipophilic nonionic surfactant such as glyceryl monostearate, propylene glycol monostearate, and sorbitan monostearate, a hydrophilic nonionic surfactant such as decaglyceryl monostearate, POE-glyceryl monoisostearate, POE-sorbitan tetraoleate, POE-behenyl ether, and coconut oil fatty acid monoethanol amide, an anionic surfactant such as sodium stearate, sodium N-stearoyl-L-glutamate, sodium POE-oleyl ether phosphate, sodium lauroyl sarcosine, and sodium lauroyl methyl alanine, a cationic surfactant such as stearyltrimethylammonium chloride, dimethylaminopropylamide stearate, and benzalkonium chloride, an amphoteric surfactant such as coconut oil fatty acid amidopropyl betaine, and betaine lauryldimethylaminoacetate, a lower alcohol such as ethanol, and isopropanol, a water-soluble macromolecule such as xanthan gum, hydroxyethyl cellulose, and carboxyvinyl polymer, a sequestering agent such as edetate disodium, a powder component, a UV absorber, an antioxidant, a pH adjustor, an organic amine, a preservative (e.g., phenoxyethanol, glycerin ethylhexyl ether, and methylparaben), a bactericide, an antiphlogistic, an astringent, a whitening agent, vitamins, amino acid, a blood circulation promoter, an activator agent, an excipient (e.g., maltitol, lactose, dextrin, and starch), a refreshing agent, various extracts, a perfume, and water. However, the present invention is not limited to these examples.

### (7) Form of foam-type external skin preparation

From the viewpoints of physical properties, use of the foam-type external skin preparation of the present invention as one preparation does not cause a problem. For example, a form in which a hydrophobically modified alkyl cellulose is contained in one preparation, a form in which a hydrophobically modified alkyl cellulose, at least one salt selected from the group consisting of a carbonate and a bicarbonate (referred to as "carbonates,"), and a water-soluble acid are contained in one preparation, and a form in which a hydrophobically modified alkyl cellulose, carbonates, a water-soluble acid, and a polyhydric alcohol are contained in one preparation. At this time, water may be contained in one preparation, or an appropriate amount of water may be added during use. In addition, the aforementioned other components may be contained.

It is preferable that the foam-type external skin preparation of the present invention be applied to a method (form) in which at least two preparations of a preparation A containing carbonates, and a preparation B containing a water-soluble acid are kept apart, and the preparations are mixed before use. At this time, a hydrophobically modified alkyl cellulose is contained in the preparation A and/or B. Further, water may be contained in the preparation A and/or B, or an appropriate amount of water may be optionally added during mixing of the preparations A and B. A polyhydric alcohol and any other components may be contained in the preparation A and/or B.

Alternatively, a method in which three preparations of a preparation A containing carbonates, a preparation B containing a water-soluble acid, and a preparation C containing a hydrophobically modified alkyl cellulose are kept apart, and the preparations are mixed before use may be applied. At this time, water may be contained in all or any of preparations A, B, and C, or may be optionally added in an appropriate amount during mixing of the preparations A, B, and C during use. A polyhydric alcohol and other components may be contained in all or any of the preparations A, B, and C.

The foam-type external skin preparation of the present invention preferably has a form in which a preparation A containing at least one salt selected from the group consisting of a carbonate and a bicarbonate and a preparation B containing a water-soluble acid are kept apart and mixed before use. At this case, the preparation A containing at least one salt selected from the group consisting of a carbonate and a bicarbonate and the preparation B containing a water-soluble acid is preferably a liquid or granular (powdered) composition.

As described above, an excellent amount and retention of carbon dioxide foam, which are not easily obtained in the conventional foam-type external skin preparation, and good usability such as prevention of the preparation sagging from the skin can be achieved by using both the preparations A and B in use.

When the foam-type external skin preparation of the present invention is prepared, it is preferable that a hydrophobically modified alkyl cellulose, carbonates, a water-soluble acid, and a polyhydric alcohol be mixed (in addition to water) and a mixture be used so that the amounts of mixed hydrophobically modified alkyl cellulose, carbonates, water-soluble acid, polyhydric alcohol, and water are 0.1 to 10.0% by mass, 0.1 to 10.0% by mass, 0.5 to 10.0% by mass, 0.1 to 50.0% by mass, and 30 to 99.3% by mass, respectively.

### (8) Aspect of use of two preparations (preparations A and B)

The foam-type external skin preparation of the present invention when two preparations (preparation A containing carbonates and preparation B containing a water-soluble acid) are used will be described.

When the foam-type external skin preparation of the present invention is used as two preparations of preparations A and B, the hydrophobically modified alkyl cellulose is contained in the preparation A and/or B. Water may be contained in the preparation A or B, or in the preparations A and B. Further, after the preparations A and B are mixed, water may be added.

The preparation A and/or B are each added so that the amount of mixed hydrophobically modified alkyl cellulose in the preparation A and/or B be preferably 0.1 to 10.0% by mass, and more preferably 0.5 to 5.0% by mass based on the whole amount of the foam-type external skin preparation. When the amount of mixed hydrophobically modified alkyl cellulose is less than 0.1% by mass based on the whole amount of the foam-type external skin preparation, an end product tends to reduce sufficient thickening effect and an effect of holding carbon dioxide (foam). When it is more than 10.0% by mass, the viscosity is too high, and mixing tends to be difficult during use. Further, application to the skin tends to be difficult. Moreover, this tends to pose a problem for production of the product.

For example, when the preparation A is a liquid composition containing 30 to 99.3% by mass of water, the content of hydrophobically modified alkyl cellulose in the preparation A is preferably 0.1 to 10% by mass, and more preferably 0.1 to 5.0% by mass. This is because the viscosity is too high to take the preparation out of a container. When the preparation A is a granular (powdered) composition, the content of hydrophobically modified alkyl cellulose in the preparation A is preferably 1 to 40% by mass, and more preferably 5 to 30% by mass. This is because the viscosity after mixing is too high, and mixing is difficult during use, and application to the skin is difficult.

When the preparation B is a liquid composition containing 30 to 99.3% by mass of water, the content of hydrophobically modified alkyl cellulose in the preparation B is preferably 0.1 to 10% by mass, and more preferably 0.1 to 5.0% by mass. This is because the viscosity is too high and the preparation is unlikely to be taken out of the container. When the preparation B is a granular (powdered) composition, the content of hydrophobically modified alkyl cellulose in the preparation B is preferably 0.1 to 10% by mass, and more preferably 0.1 to 5.0% by mass. This is because the viscosity after mixing is too high, mixing is difficult during use, and application to the skin is difficult.

The preparation A and/or B may be a liquid composition containing water. When the preparations A and B are granular (powdered) compositions, water is added during use so that the amount is preferably 300 to 99,300 parts by mass, and more preferably 1,140 to 17,700 parts by mass based on 100 parts by mass of hydrophobically modified alkyl cellulose contained in the whole amount of the foam-type external skin preparation.

The content of at least one salt selected from the group consisting of a carbonate and a bicarbonate (carbonates) in the preparation A is preferably 0.1 to 10.0% by mass, and more preferably 0.5 to 5.0% by mass based on the whole amount of the foam-type external skin preparation such as end product. When the content is less than 0.1% by mass, foaming due to carbon dioxide is not sufficiently obtained during use. When it is more than 10.0% by mass, a large amount of carbon dioxide is generated, and the preparation may irritate the skin during use. When the preparation A is a liquid or granular or powdered composition, carbonates in the amount within the range may be contained.

The content of water-soluble acid in the preparation B is preferably 1.0 to 80.0% by mass. The content of water-soluble acid in the preparation B is preferably 0.5 to 10.0% by mass based on the whole amount of the foam-type external skin preparation such as an end product. Further, the mixed amount is particularly preferably such an amount that the pH of the foam-type external skin preparation after mixing with the preparation A is 3.0 to 8.0. When the pH is lower than 3.0, the preparation may irritate the skin during use. When the pH is higher than 8.0, the amount of generated carbon dioxide is not sufficient due to very mild degradation of carbonates. When the content of water-soluble acid in the preparation B is less than 1.0% by mass and the addition amount is more than 80.0% by mass, it is difficult that the pH is adjusted to the range described herein. When the preparation B is a liquid or granular or powdered composition, a water-soluble acid in an amount within the range may be contained.

When the foam-type external skin preparation of the present invention is used as two preparations of preparations A and B, there are no problem in terms of physical properties although the polyhydric alcohol is contained in the preparation A and/or B.

The preparation A and/or B are each mixed so that the amount of mixed polyhydric alcohol in the preparation A and/or B is preferably 0.1 to 50.0% by mass, and more preferably 10.0 to 30.0% by mass based on the whole amount of the foam-type external skin preparation. When the amount of mixed polyhydric alcohol based on the whole amount of foam-type external skin preparation is less than 0.1% by mass, the physical properties of thickening composition are not uniform (lumps), and a mixing process during use and application to the skin are difficult. When it is more than 50.0% by mass, stickiness is increased during use. Therefore, the sense of use is not good.

When the foam-type external skin preparation of the present invention is prepared, it is preferable that appropriate amounts of preparations A and B (in addition to water) be mixed and used so that the amounts of mixed hydrophobically modified alkyl cellulose, carbonates, water-soluble acid, polyhydric alcohol, and water are 0.1 to 10.0% by mass, 0.1 to 10.0% by mass, 0.5 to 10.0% by mass, 0.1 to 50.0% by mass, and 30 to 99.3% by mass, respectively.

### (9) Aspect of use of three preparations (preparations A, B, and C)

The foam-type external skin preparation of the present invention when three preparations (preparation A containing carbonates, preparation B containing water-soluble acid, and preparation C containing hydrophobically modified alkyl cellulose) are used will be described. Water may be contained in any or all of the preparations A, B, and C. Further, after the preparations A, B, and C are mixed, water may be added.

When the preparation A is a liquid composition containing 30 to 99.3% by mass of water, the content of carbonates in the preparation A is preferably 0.1 to 10.0% by mass, and more preferably 0.5 to 5.0% by mass. This allows obtaining sufficient foaming due to carbon dioxide, preventing an excessively large amount of foaming, and reducing irritation to the skin during use. When the preparation A is a granular (powdered) composition, the content of carbonates in the preparation A may be adjusted to be preferably 0.1 to 10.0% by mass, and more preferably 0.5 to 5.0% by mass based on the whole amount of the foam-type external skin preparation such as an end product from the same reasons.

When the preparation B is a liquid composition containing 30 to 99.3% by mass of water, the content of water-soluble acid in the preparation B is preferably 0.5 to 10.0% by mass. When the preparation B is a granular (powdered) composition, the content of water-soluble acid in the preparation B is preferably 0.5 to 50% by mass. The amount of water-soluble acid to be mixed is preferably 0.1 to 10.0% by mass based on the whole amount of foam-type external skin preparation such as an end product after mixing of the preparation A and a preparation C described below, and particularly preferably such an amount that the pH of foam-type external skin preparation is 3.0 to 8.0. When the pH is lower than 3.0, the preparation may irritate the skin in use. When the pH is higher than 8.0, the amount of generated carbon dioxide gas is not sufficient due to very mild degradation of carbonates. When the content of water-soluble acid in the preparation B is an addition amount without the range of the content, the pH is unlikely to be adjusted to the range described herein.

When the preparation C is a liquid composition containing 30 to 99.3% by mass of water, the content of hydrophobically modified alkyl cellulose in the preparation C is preferably 0.1 to 10% by mass, and more preferably 0.5 to 5.0% by mass. When the preparation C is a granular (powdered) composition, the content of hydrophobically modified alkyl cellulose in the preparation C is preferably 0.1 to 10.0% by mass, and more preferably 0.5 to 5.0% by mass. Alternatively, the content of hydrophobically modified alkyl cellulose may be adjusted to be 0.1 to 10.0% by mass based on the whole amount of the foam-type external skin preparation such as an end product. When the amount of mixed hydrophobically modified alkyl cellulose falls within the range, an end product can have sufficient thickening effect and an effect of holding carbon dioxide gas (foam). Further, the viscosity is not too high, and mixing is easy in use. Moreover, application to the skin is easy, and therefore this is preferable for production of the product.

When the foam-type external skin preparation of the present invention is used as three preparations of the preparations A, B, and C, there are no problem in terms of physical properties although the polyhydric alcohol is contained in all of the preparations A, B, and C. Each of preparation A, B, and/or C is mixed so that the amount of mixed polyhydric alcohol is preferably 0.1 to 50.0% by mass, and more preferably 10.0 to 30.0% by mass based on the whole amount of the foam-type external skin preparation.

When the amount of mixed polyhydric alcohol falls within the range, the physical properties of thickening composition in the end product are uniformly maintained (no lumps), and a mixing process during use and application to the skin are suitably performed. Further, stickiness is not caused during use and the sense of use can be improved.

When the preparations A, B, and C are granular (powdered) compositions, water is added during use so that the amount is preferably 1.0 to 50,000 parts by mass, and more preferably 200 to 6,000 parts by mass based on 100 parts by mass of hydrophobically modified alkyl cellulose contained in the whole amount of the foam-type external skin preparation.

When the foam-type external skin preparation of the present invention is prepared, it is preferable that appropriate amounts of preparations A, B, and C (in addition to water) be mixed and used so that the amounts of mixed hydrophobically modified alkyl cellulose, carbonates, water-soluble acid, polyhydric alcohol, and water are 0.1 to 10.0% by mass, 0.1 to 10.0% by mass, 0.5 to 10.0% by mass, 0.1 to 50.0% by mass, and 30 to 99.3% by mass, respectively.

### (10) Specific examples of aspect of use of foam-type external skin preparation

Hereinafter, preferable examples of the foam-type external skin preparation of the present invention when the preparation A containing carbonates and the preparation B containing a water-soluble acid are used will be specifically described.

### (A) Preparation A is liquid composition, and preparation B is granular (powdered) composition

A hydrophobically modified alkyl cellulose is contained in a preparation A and/or B.

### [Preparation A]

- Water: 40.0 to 90.0% by mass (preferably 70 to 85% by mass)
- Hydrophobically modified alkyl cellulose (e.g., SANGELOSE 60L available from Daido Chemical Corporation.) (when it is mixed in a preparation A): 0.1 to 10.0% by mass (preferably 0.5 to 3% by mass)
- At least one salt selected from the group consisting of a carbonate and a bicarbonate (e.g., sodium bicarbonate, and sodium carbonate): 0.1 to 5.0% by mass (preferably 0.5 to 4% by mass)
- Polyhydric alcohol (e.g., 1,3-butylene glycol, 1,2-pentanediol, or other components): 0.1 to 50.0% by mass (preferably 10 to 30% by mass)

### [Preparation B]

- Hydrophobically modified alkyl cellulose (e.g., SANGELOSE 60L available from Daido Chemical Corporation.) (when it is mixed in a preparation B): 0.1 to 10.0% by mass (preferably 0.5 to 3% by mass)
- Water-soluble acid (e.g., citric acid, glycolic acid, malic acid, and succinic acid): 20.0 to 80.0% by mass (preferably 25 to 55% by mass)
- Excipient (e.g., maltitol, lactose, and gluconolactone): 20.0 to 80.0% by mass

The preparations A and B are mixed immediately before use.

The mixing ratio (by mass) of the preparations A and B may be, not particularly limited to, adjusted based on the amounts of mixed components in the preparations A and B. It is preferable that the preparations A and B be generally used in a mixed ratio of 30:0.1 to 10. It is further preferable that the ratio of mixed preparations A and B be, for example, 30:1, 30:3, 30:5, or 30:7

### (B) Preparation A is liquid composition, and Preparation B is liquid composition

A hydrophobically modified alkyl cellulose is contained in a preparation A and/or B.

### [Preparation A]

- Water: 40.0 to 90.0% by mass (preferably 70 to 85% by mass)
- Hydrophobically modified alkyl cellulose (e.g., SANGELOSE 60L available from Daido Chemical Corporation.) (when it is mixed in a preparation A): 0.1 to 10.0% by mass (preferably 0.5 to 3% by mass)
- At least one salt selected from the group consisting of a carbonate and a bicarbonate (e.g., sodium bicarbonate, and sodium carbonate): 0.1 to 10.0% by mass (preferably 0.5 to 5.0% by mass)
- Polyhydric alcohol (e.g., 1,3-butylene glycol, 1,2-pentanediol, or other components): 0.1 to 50.0% by mass (preferably 10 to 30% by mass)

### [Preparation B]

- Water: 40.0 to 90.0% by mass (preferably 40 to 80% by mass)
- Hydrophobically modified alkyl cellulose (e.g., SANGELOSE 60L available from Daido Chemical Corporation.) (when it is mixed in a preparation B): 0.1 to 10.0% by mass (preferably 0.5 to 3% by mass)
- Water-soluble acid (e.g., citric acid, glycolic acid, malic acid, and succinic acid): 1 to 80.0% by mass (preferably 1.5 to 60% by mass)
- Excipient (e.g., maltitol, lactose, and gluconolactone): 20.0 to 80.0% by mass

The preparations A and B are mixed immediately before use.

The preferable mixing ratio (by mass) of the preparations A and B is not particularly limited, but the preparations A and B are preferably used in a mixed ratio of 30:1 to 45. For example, the ratio of mixed preparations A and B may be 1:1.

### (C) Preparation A is granular (powdered) composition, and Preparation B is liquid composition

A hydrophobically modified alkyl cellulose is contained in a preparation A and/or B.

### [Preparation A]

- Hydrophobically modified alkyl cellulose (e.g., SANGELOSE 60L available from Daido Chemical Corporation.) (when it is mixed in a preparation A): 0.1 to 30% by mass (preferably 20 to 30% by mass)
- At least one salt selected from the group consisting of a carbonate and a bicarbonate (e.g., sodium bicarbonate, and sodium carbonate): 50 to 90% by mass (preferably 65 to 80% by mass)
- Polyhydric alcohol (e.g., 1,3-butylene glycol, 1,2-pentanediol, or other components): 0.1 to 50.0% by mass

### [Preparation B]

- Water: 40.0 to 90.0% by mass (preferably 40 to 80% by mass)
- Hydrophobically modified alkyl cellulose (e.g., SANGELOSE 60L available from Daido Chemical Corporation.) (when it is mixed in a preparation B): 0.1 to 10.0% by mass (preferably 0.5 to 3% by mass)
- Water-soluble acid (e.g., citric acid, glycolic acid, malic acid, and succinic acid): 1 to 80.0% by mass (preferably 1.5 to 60% by mass)
- Excipient (e.g., maltitol, lactose, and gluconolactone): 20.0 to 80.0% by mass

The preparations A and B are mixed immediately before use.

The preferable mixing ratio (by mass) of the preparations A and B is not particularly limited, but the preparations A and B are preferably used in a mixing ratio of 1:2 to 300. It is further preferable that the mixing ratio of the preparations A and B be 1:30,1:10,1:3, or 1:2.

The foam-type external skin preparation of the present invention is not limited to sites of application, and can be used as a cosmetic material for body (e.g., scalp hair grower) to promote the circulation of blood in the whole body (scalp). Further, the foam-type external skin preparation is preferably used for the face as a skin care cosmetic material having a high effect by direct action. The foam-type external skin preparation can be used as a skin care cosmetic material in a dosage form such as a lotion, an emulsion, a cream, an ointment, and a sheet. A pack cosmetic material is most preferable since it can coat the face and trap carbon dioxide gas to increase the concentration, and thus the effect of promoting the circulation of blood can be enhanced.

The foam-type external skin preparation of the present invention mainly represents a skin care preparation typified by a cosmetic pack, and can be used as a clinical therapy preparation.

A method for promoting the circulation of blood in the whole body (scalp) and a method for promoting vasodilation in the whole body (scalp) using the foam-type external skin preparation of the present invention are preferable.

Hereinafter, the present invention will be described in details with reference to Examples. However the present invention is not limited to these examples. Examples

The form of preparation of the present invention and the effect obtained from it will be specifically described with reference to Examples and Comparative Examples.

### Example 1

Foam-type pack preparations A and B shown below (Table 1: composition) were prepared. The obtained foam-type pack preparations were mixed in a ratio of the preparation A to the preparation B of 30 g to 1.0 g. Foaming during mixing was confirmed and foam retention was evaluated. In addition, the presence or absence of liquid sagging from the buccal region when the preparation was actually used on the buccal region was evaluated. The results of the evaluation are shown below (Table 2: results). The evaluation was visually performed and judged in accordance with the following criteria.

### [Foaming during mixing]

Excellent: Foaming is significantly confirmed.
Good: Foaming is confirmed at the initiation of mixing, but a decrease of foam caused by release in the air is confirmed during mixing.
Poor: Foaming is confirmed at the initiation of mixing, but most of foam is released in the air during mixing.

### [Foam retention]

Excellent: There is no change from the initial state (immediately after mixing) (foam does not disappear).
Good: A slight decrease appears from the initial state, but sufficiently remained foam is confirmed.
Poor: A decrease of foam is clearly confirmed.

### [Presence or absence of liquid sagging during use]

Excellent: Liquid sagging does not occur.
Good: Liquid sagging occurs with time.
Poor: Liquid sagging occurs immediately after coating.

**[Table 1]**

| | Name of component | Example 1 Component configuration (% by mass) | Comparative Example 1 Component configuration (% by mass) | Comparative Example 2 Component configuration (% by mass) |
|---|---|---|---|---|
| | Water | 82.5 | 82.5 | 82.5 |
| | Hydroxypropyl methylcellulose stearoxy ether | 1.0 | - | - |
| Preparation A | Carboxymethyl cellulose | - | 1.0 | - |
| | Sodium alginate | - | - | 1.0 |
| | 1,3-Butylene glycol | 15.0 | 15.0 | 15.0 |
| | Sodium bicarbonate | 1.0 | 1.0 | 1.0 |
| | Phenoxyethanol | 0.5 | 0.5 | 0.5 |
| Preparation B | Citric acid | 50.0 | 50.0 | 50.0 |
| | Maltitol | 50.0 | 50.0 | 50.0 |

In Table 1, hydroxypropyl methylcellulose stearoxy ether (trade name: SANGELOSE 60L, available from Daido Chemical Corporation.), carboxymethylcellulose (trade name: CMC DAICEL 1380, available from Daicel FineChem Ltd.), sodium alginate (trade name: Duck Algin NSPH available from Kibun Food Chemifa Co., Ltd.), 1,3-butylene glycol (trade name: 1,3-BGK, available from Daicel Corporation.), sodium bicarbonate (trade name: sodium bicarbonate (Japanese pharmacopoeia), available from TOSOH CORPORATION), phenoxyethanol (trade name: Hisolve EPH, available from TOHO Chemical Industry Co., Ltd.), citric acid (trade name: citric acid (crystal), available from IWATA CHEMICAL, LTD.), and maltitol (trade name: powder MABIT, available from HAYASHIBARA CO., LTD.) were used.

**[Table 2]**

| Evaluation criteria | Example 1 | Comparative Example 1 | Comparative Example 2 |
|---|---|---|---|
| Foaming during mixing | 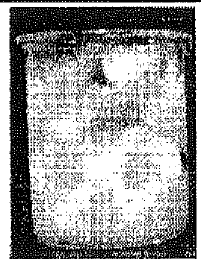 | 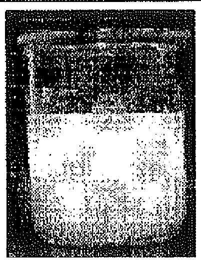 | 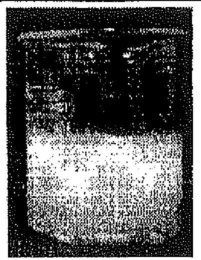 |
| Height of foam (mm) | 37 | 38 | 24 |
| Evaluation results | (Excellent) | (Excellent) | (Good) |
| Foam retention (after five minutes) | 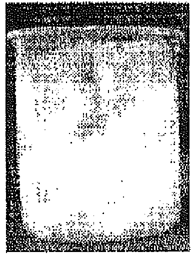 | 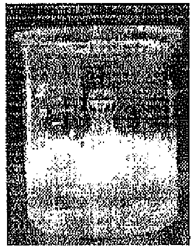 | 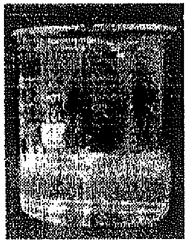 |
| Height of foam (mm) | 35 | 18 | 3 |
| Evaluation results | (Excellent) | (Poor) | (Poor) |
| Foam retention (after ten minutes) | 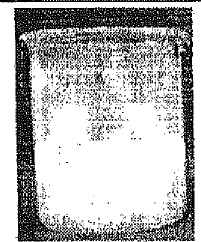 | 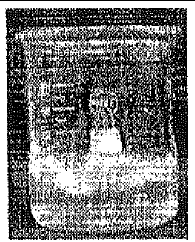 | 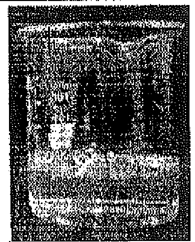 |
| Height of foam (mm) | 35 | 6 | 2 |
| Evaluation results | (Excellent) | (Poor) | (Poor) |
| Presence or absence of liquid sagging | (Excellent) | (Poor) | (Poor) |

As seen from Table 2, foaming during mixing and foam retention are excellent in Example 1, and sagging from the bucca does not occur in use due to elasticity of foam. On the other hand, foaming and foam retention are insufficient in Comparative Examples 1 and 2, and sagging from the bucca occurs in use.

### Example 2

Foam-type pack preparations A and B having such a high viscosity that liquid sagging is not caused, shown below (Table 3: composition), were prepared. The obtained foam-type pack preparations were mixed in a ratio of the preparation A to the preparation B of 30 g to 1.0 g. Foaming during mixing was confirmed and foam retention was evaluated. In addition, the presence or absence of liquid sagging from the buccal region when the preparation was actually used on the buccal region was evaluated. The results of the evaluation are shown below (Table 4: results). The evaluation was visually performed and judged in accordance with the following criteria.

### [Foaming during use]

Excellent: Foaming is significantly confirmed.
Good: Foaming is confirmed, but the reaction is mild, and release in the air is confirmed during mixing.
Poor: Foaming is confirmed at the initiation of mixing, but most of foam is released in the air during mixing.

### [Foam retention]

Excellent: There is no change from the initial state (immediately after mixing) (foam does not disappear).
Good: A slight decrease from the initial state appears, but sufficiently remained foam is confirmed.
Poor: A decrease of foam is clearly confirmed.

### [Presence or absence of liquid sagging during use]

Excellent: Liquid sagging does not occur.
Good: Liquid sagging occurs with time.
Poor: Liquid sagging occurs immediately after coating.

**[Table 3]**

| | Name of component | Example 2 Component configuration (% by mass) | Comparative Example 3 Component configuration (% by mass) |
|---|---|---|---|
| Preparation A | Water | 81.0 | 79.5 |
| | Hydroxypropyl methylcellulose stearoxy ether | 2.5 | - |
| | Sodium alginate | - | 4.0 |
| | 1,3-Butylene glycol | 14.0 | 14.0 |
| | Sodium bicarbonate | 1.5 | 1.5 |
| | 1,2-pentanediol | 1.0 | 1.0 |
| Preparation B | Water | 49.5 | 49.5 |
| | Citric acid | 50.0 | 50.0 |
| | Phenoxyethanol | 0.5 | 0.5 |

In Table 3, 1,2-pentanediol (trade name: Diol PD, available from KOKYU ALCOHOL KOGYO CO., LTD.) was used.

**[Table 4]**

| Evaluation criteria | Example 2 | Comparative Example 3 |
|---|---|---|
| Foaming during mixing | 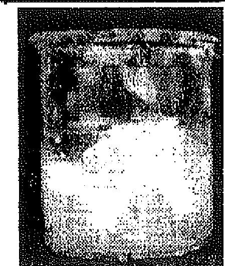 | 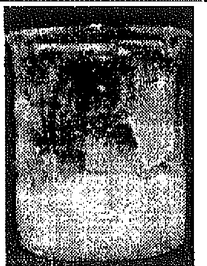 |
| Height of foam (mm) | 42 | 25 |
| Evaluation results | (Excellent) | (Good) |
| Foam retention (after five minutes) | 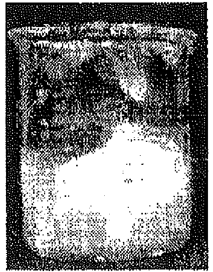 | 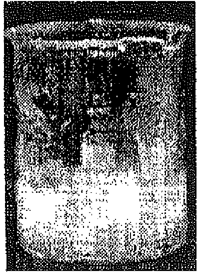 |
| Height of foam (mm) | 36 | 18 |
| Evaluation results | (Excellent) | (Good) |
| Foam retention (after ten minutes) | 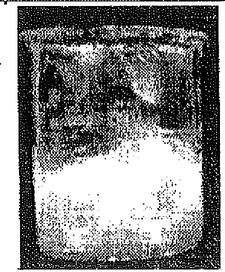 | 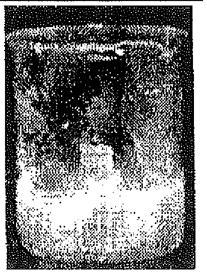 |
| Height of foam (mm) | 35 | 16 |
| Evaluation results | (Excellent) | (Good) |
| Presence or absence of liquid sagging | (Excellent) | (Excellent) |

As seen from Table 4, foaming during mixing and foam retention are excellent in Example 2. Further, sufficient viscosity is maintained in use, and sagging from the bucca does not occur. On the other hand, in Comparative Example 3, liquid sagging from the bucca does not occur during use, but foaming and foam retention are insufficient during mixing.

### Example 3

Foam-type pack preparations A and B having such a high viscosity that liquid sagging is not caused, shown below (Table 5: composition), were prepared (Examples 3-1 to 3-5). In the composition, the preparation A (containing carbonates) contains water and hydroxypropyl methylcellulose stearoxy ether. The obtained foam-type pack preparations were mixed in a ratio of the preparation A to the preparation B of 30 g to 5.0 g. Foaming during mixing was confirmed and foam retention was evaluated. In addition, the presence or absence of liquid sagging from the buccal region when the preparation was actually used on the buccal region was evaluated.

**[Table 5]**

| | Name of component | Example 3-1 Component configuration (% by mass) | Example 3-2 Component configuration (% by mass) | Example 3-3 Component configuration (% by mass) | Example 3-4 Component configuration (% by mass) | Example 3-5 Component configuration (% by mass) |
|---|---|---|---|---|---|---|
| Preparation A | Water | 75.5 | 75.5 | 75.5 | 75.5 | 75.5 |
| | Hydroxypropyl methylcellulose stearoxy ether | 2.5 | 2.5 | 2.5 | 2.5 | 2.5 |
| | 1,3-Butylene glycol | 20.0 | 20.0 | 20.0 | 20.0 | 20.0 |
| | Sodium bicarbonate | 1.5 | - | 1.5 | 1.5 | 1.5 |
| | Sodium carbonate | - | 1.5 | - | - | - |
| | Dipotassium glycyrrhizate | 0.1 | 0.1 | 0.1 | 0.1 | 0.1 |
| | Tetrasodium edetate | 0.1 | 0.1 | 0.1 | 0.1 | 0.1 |
| | Phenoxyethanol | 0.3 | 0.3 | 0.3 | 0.3 | 0.3 |
| Preparation B | Citric acid | 30.0 | 30.0 | - | - | 20.0 |
| | Malic acid | - | - | 30.0 | - | - |
| | Succinic acid | - | - | - | 30.0 | - |
| | Glucono delta lactone | - | - | - | - | 80.0 |
| | Maltitol | 70.0 | 70.0 | 70.0 | 70.0 | - |

In Table 5, sodium carbonate (trade name: sodium carbonate, available from TOSOH CORPORATION), dipotassium glycyrrhizate (trade name: dipotassium glycyrrthin K2 available from MARUZEN PHARMACEUTICALS CO., LTD.), tetrasodium edetate (trade name: Clewat T, available from Nagase ChemteX Corporation), malic acid (trade name: Malic Acid FUSO, available from FUSO CHEMICAL CO., LTD.), succinic acid (trade name: succinic acid, available from FUSO CHEMICAL CO., LTD.), and glucono delta lactone (trade name: Riken lactone, available from Riken Vitamin Co., Ltd.) were used.

Foaming during mixing and foam retention are excellent in Examples 3-1 to 3-5, similar to Example 1. Further, sufficient viscosity is maintained during use, and sagging from the bucca does not occur.

### Example 4

Foam-type pack preparations A and B having such a high viscosity that sagging is not caused, shown below (Table 6: composition), were prepared (Examples 4-1 to 4-5). In the composition, the preparation A (containing carbonates) and the preparation B (containing water-soluble acid) contain hydroxypropyl methylcellulose stearoxy ether, and the preparation B contains water. The obtained foam-type pack preparations were mixed in a ratio of the preparation A to the preparation B of 2.0 g to 20.0 g. Foaming during mixing was confirmed and foam retention was evaluated. In addition, the presence or absence of liquid sagging from the buccal region when the preparation was actually used on the buccal region was evaluated.

**[Table 6]**

| | Name of component | Example 4-1 Component configuration (% by mass) | Example 4-2 Component configuration (% by mass) | Example 4-3 Component configuration (% by mass) | Example 4-4 Component configuration (% by mass) | Example 4-5 Component configuration (% by mass) |
|---|---|---|---|---|---|---|
| Preparation A | Sodium bicarbonate | 75.0 | - | 75.0 | 75.0 | 75.0 |
| | Sodium carbonate | - | 75.0 | - | - | - |
| | Hydroxypropyl methylcellulose stearoxy ether | 25.0 | 25.0 | 25.0 | 25.0 | 25.0 |
| | Water | 60.0 | 60.0 | 60.0 | 60.0 | 55.0 |
| | Hydroxypropyl methylcellulose stearoxy ether | 1.5 | 1.5 | 1.5 | 1.5 | 1.5 |
| | 1,3-Butylene glycol | 30.0 | 30.0 | 30.0 | 30.0 | 30.0 |
| | Glycerin | 3.0 | 3.0 | 3.0 | 3.0 | 3.0 |
| Preparation B | Citric acid | 5.0 | 5.0 | - | - | - |
| | Malic acid | - | - | 5.0 | - | - |
| | Succinic acid | - | - | - | 5.0 | - |
| | Glucono delta lactone | - | - | - | - | 10.0 |
| | Xanthan gum | 0.3 | 0.3 | 0.3 | 0.3 | 0.3 |
| | Methyl parahydroxybenzoate | 0.2 | 0.2 | 0.2 | 0.2 | 0.2 |

In Table 6, glycerin (trade name: concentrated glycerin for cosmetics, available from SAKAMOTO YAKUHIN KOGYO CO., LTD.), xanthan gum (trade name: KELTROL, available from Sansho Co., Ltd.), and methyl parahydroxybenzoate (trade name: MEKKINS M, available from UENO FINE CHEMICALS INDUSTRY, LTD.) were used.

Foaming during mixing and foam retention are excellent in Examples 4-1 to 4-5, similar to Example 1. Further, sufficient viscosity is maintained during use, and sagging from the bucca does not occur.

### Example 5

Foam-type pack preparations A and B having such a high viscosity that liquid sagging is not caused, shown below (Table 7: composition), were prepared (Examples 5-1 to 5-5). In the composition, the preparation A (containing carbonates) and the preparation B (containing water-soluble acid) each contain hydroxypropyl methylcellulose stearoxy ether and water. The obtained foam-type pack preparations were mixed in a ratio of the preparation A to the preparation B of 10.0 g to 10.0 g. Foaming during mixing was confirmed and foam retention was evaluated. In addition, the presence or absence of liquid sagging from the buccal region when the preparation was actually used on the buccal region was evaluated.

**[Table 7]**

| | Name of component | Example 5-1 Component configuration (% by mass) | Example 5-2 Component configuration (% by mass) | Example 5-3 Component configuration (% by mass) | Example 5-4 Component configuration (% by mass) | Example 5-5 Component configuration (% by mass) |
|---|---|---|---|---|---|---|
| Preparation A | Water | 74.0 | 74.0 | 74.0 | 74.0 | 74.0 |
| | Hydroxypropyl methylcellulose stearoxy ether | 0.8 | 0.8 | 0.8 | 0.8 | 0.8 |
| | 1,3-Butylene glycol | 20.0 | 20.0 | 20.0 | 20.0 | 20.0 |
| | Sodium bicarbonate | 3.0 | - | 3.0 | 3.0 | 3.0 |
| | Sodium carbonate | - | 3.0 | - | - | - |
| | 1,2-Pentanediol | 2.0 | 2.0 | 2.0 | 2.0 | 2.0 |
| | Polyoxyethylene hydrogenated castor oil (60E.O.) | 0.15 | 0.15 | 0.15 | 0.15 | 0.15 |
| | Tocopherol | 0.05 | 0.05 | 0.05 | 0.05 | 0.05 |
| Preparation B | Water | 77.0 | 77.0 | 77.0 | 77.0 | 74.0 |
| | Hydroxypropyl methylcellulose stearoxy ether | 1.0 | 1.0 | 1.0 | 1.0 | 1.0 |
| | 1,3-Butylene glycol | 20.0 | 20.0 | 20.0 | 20.0 | 20.0 |
| | Citric acid | 2.0 | 2.0 | - | - | - |
| | Malic acid | - | - | 2.0 | - | - |
| | Succinic acid | - | - | - | 2.0 | - |
| | Glucono delta lactone | - | - | - | - | 5.0 |

Foaming during mixing and foam retention are excellent in Examples 5-1 to 5-5, similar to Example 2. Further, sufficient viscosity is maintained during use, and sagging from the bucca does not occur.

## Claims

1. A foam-type external skin preparation comprising: a hydrophobically modified alkyl cellulose; at least one salt selected from the group consisting of a carbonate and a bicarbonate; and a water-soluble acid, wherein the hydrophobically modified alkyl cellulose is represented by the following structural formula (1): 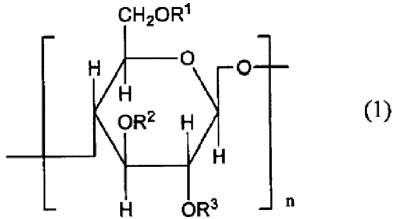 wherein R¹, R², and R³ are the same or different, and are a hydrogen atom, a lower alkyl group, a -[CH₂CH₂₋ₖ(CH₃)ₖO]ₘH group, or a -CH₂CH(OH)CH₂OCⱼH₂ⱼ₊₁ group; n is an integer of 100 to 10,000; k is an integer of 0 or 1; m is an integer of 1 to 10; and j is an integer of 6 to 26; and the hydrophobically modified alkyl cellulose necessarily contains a -CH₂CH(OH)CH₂OCⱼH₂ⱼ₊₁ group;
wherein the lower alkyl group is a methyl group or an ethyl group.

2. The foam-type external skin preparation according to claim 1, wherein the hydrophobically modified alkyl cellulose represented by the structural formula (1) contains 10.0 to 50.0% by mass of a lower alkyl group, 3.0 to 20.0% by mass of a -[CH₂CH₂₋ₖ(CH₃)ₖO]ₘH group, and 0.1 to 10.0% by mass of a -CH₂CH(OH)CH₂OCⱼH₂ⱼ₊₁ group.

3. The foam-type external skin preparation according to claim 1 or 2, wherein the value j of the -CH₂CH(OH)CH₂OCⱼH₂ⱼ₊₁ group in the structural formula (1) is 18.

4. The foam-type external skin preparation according to any one of claims 1 to 3, wherein the at least one salt selected from the group consisting of a carbonate and a bicarbonate is sodium bicarbonate.

5. The foam-type external skin preparation according to any one of claims 1 to 4, wherein the water-soluble acid is a hydroxy acid.

6. The foam-type external skin preparation according to claim 5, wherein the hydroxy acid is citric acid.

7. The foam-type external skin preparation according to any one of claims 1 to 6, further comprising a polyhydric alcohol.

8. The foam-type external skin preparation according to claim 7, wherein the polyhydric alcohol contains one or more members selected from the group consisting of propylene glycol, dipropylene glycol, 1,3-butylene glycol, and 1,2-pentanediol.

9. The foam-type external skin preparation according to any one of claims 1 to 8, wherein the foam-type external skin preparation comprises a preparation A containing at least one salt selected from the group consisting of a carbonate and a bicarbonate and a preparation B containing a water-soluble acid, the preparation A and preparation B having been kept apart and mixed before use.

10. A method for preparing a foam-type external skin preparation, wherein the foam-type external skin preparation comprises: a hydrophobically modified alkyl cellulose; at least one salt selected from the group consisting of a carbonate and a bicarbonate; and a water-soluble acid; wherein the hydrophobically modified alkyl cellulose is represented by the following structural formula (1): 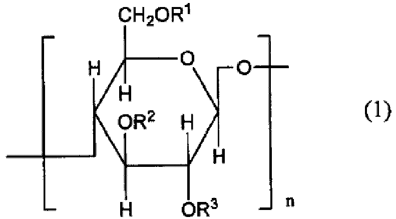 wherein R¹, R² and R³ are the same or different, and are a hydrogen atom, a lower alkyl group a -[CH₂CH₂₋ₖ(CH₃)ₖO]ₘH group, or a -CH₂CH(OH)CH₂OCⱼH₂ⱼ₊₁ group; n is an integer of 100 to 10,000; k is an integer of 0 or 1; m is an integer of 1 to 10; and j is an integer of 6 to 26; and the hydrophobically modified alkyl cellulose necessarily contains a -CH₂CH(OH)CH₂OCⱼH₂ⱼ₊₁ group, wherein the lower alkyl group is a methyl group or an ethyl group;
the method comprising:
mixing before use a preparation A containing at least one salt selected from the group consisting of a carbonate and bicarbonate and a preparation B containing a water-soluble acid, the preparation A and the preparation B having been kept apart.

11. A method of use of a foam-type external skin preparation, wherein the foam type external preparation comprises: a hydrophobically modified alkyl cellulose; at least one salt selected from the group consisting of a carbonate and bicarbonate; and a water-soluble acid, wherein the hydrophobically modified alkyl cellulose is represented by the following structural formula (1): 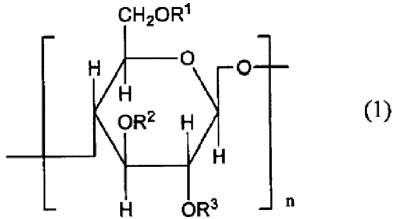 wherein R¹, R² and R³ are the same or different, and are a hydrogen atom, a lower alkyl group a -[CH₂CH₂₋ₖ(CH₃)ₖO]ₘH group, or a -CH₂CH(OH)CH₂OCⱼH₂ⱼ₊₁ group; n is an integer of 100 to 10,000; k is an integer of 0 or 1; m is an integer of 1 to 10; and j is an integer of 6 to 26; and the hydrophobically modified alkyl cellulose necessarily contains a -CH₂CH(OH)CH₂OCⱼH₂ⱼ₊₁ group, wherein the lower alkyl group is a methyl group or an ethyl group; and wherein the foam-type external skin preparation comprises a preparation A containing at least one salt selected from the group consisting of a carbonate and a bicarbonate and a preparation B containing a water-soluble acid, the preparation A and the preparation B having been kept apart and mixed before use.

12. The foam-type external skin preparation according to any one of claims 1-9, wherein the foam-type external skin preparation is a pack cosmetic material used for the face.

## Patentansprüche

1. Schaumartiges externes Hautpräparat umfassend: eine hydrophobisch modifizierte Alkylcellulose; mindestens ein Salz ausgewählt aus der Gruppe bestehend aus einem Carbonat und einem Bicarbonat; und eine wasserlösliche Säure, wobei die hydrophobisch modifizierte Alkylcellulose durch die folgende strukturelle Formel (1) dargestellt ist: 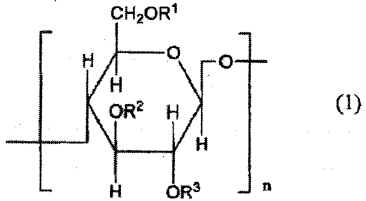 wobei R¹, R² und R³ gleich oder verschieden sind und ein Wasserstoffatom, eine niedere Alkylgruppe, eine -[CH₂CH₂₋ₖ(CH₃)ₖO]ₘH-Gruppe oder eine - CH₂CH(OH)CH₂OCⱼH₂ⱼ₊₁-Gruppe sind; n eine ganze Zahl von 100 bis 10000 ist; k eine ganze Zahl von 0 oder 1 ist; m eine ganze Zahl von 1 bis 10 ist; und j eine ganze Zahl von 6 bis 26 ist; und die hydrophobisch modifizierte Alkylcellulose notwendigerweise eine - CH₂CH(OH)CH₂OCⱼH₂ⱼ₊₁-Gruppe enthält;
wobei die niedere Alkylgruppe eine Methylgruppe oder eine Ethylgruppe ist.

2. Schaumartiges externes Hautpräparat nach Anspruch 1, wobei die hydrophobisch modifizierte Alkylcellulose, die durch die strukturelle Formel (1) dargestellt ist, 10,0 bis 50,0 Masse-% einer niederen Alkylgruppe, 3,0 bis 20,0 Masse-% einer -[CH₂CH₂₋ₖ(CH₃)ₖO]ₘH-Gruppe und 0,1 bis 10,0 Masse-% einer -CH₂CH(OH)CH₂OCⱼH₂ⱼ₊₁-Gruppe enthält.

3. Schaumartiges externes Hautpräparat nach Anspruch 1 oder 2, wobei der Wert von j der -CH₂CH(OH)CH₂OCⱼH₂ⱼ₊₁-Gruppe in der strukturellen Formel (1) 18 beträgt.

4. Schaumartiges externes Hautpräparat nach irgendeinem der Ansprüche 1 bis 3, wobei das mindestens eine Salz, das aus der Gruppe ausgewählt wird, die aus einem Carbonat und einem Bicarbonat besteht, Natriumbicarbonat ist.

5. Schaumartiges externes Hautpräparat nach irgendeinem der Ansprüche 1 bis 4, wobei die wasserlösliche Säure eine Hydroxysäure ist.

6. Schaumartiges externes Hautpräparat nach Anspruch 5, wobei die Hydroxysäure Zitronensäure ist.

7. Schaumartiges externes Hautpräparat nach irgendeinem der Ansprüche 1 bis 6, ferner einen mehrwertigen Alkohol umfassend.

8. Schaumartiges externes Hautpräparat nach Anspruch 7, wobei der mehrwertige Alkohol eine oder mehrere Teilnehmersubstanzen enthält ausgewählt aus der Gruppe bestehend aus Propylenglykol, Dipropylenglykol, 1,3-Butylenglykol und 1,2-Pentandiol.

9. Schaumartiges externes Hautpräparat nach irgendeinem der Ansprüche 1 bis 8, wobei das schaumartige externe Hautpräparat ein Präparat A, das mindestens ein Salz enthält ausgewählt aus der Gruppe bestehend aus einem Carbonat und einem Bicarbonat, und ein Präparat B umfasst, das eine wasserlösliche Säure enthält, wobei das Präparat A und das Präparat B getrennt gehalten und vor der Verwendung gemischt werden.

10. Verfahren zum Herstellen eines schaumartigen externen Hautpräparats, wobei das schaumartige externe Hautpräparat Folgendes umfasst: eine hydrophobisch modifizierte Alkylcellulose; mindestens ein Salz ausgewählt aus der Gruppe bestehend aus einem Carbonat und einem Bicarbonat; und eine wasserlösliche Säure; wobei die hydrophobisch modifizierte Alkylcellulose durch die folgende strukturelle Formel (1) dargestellt ist: 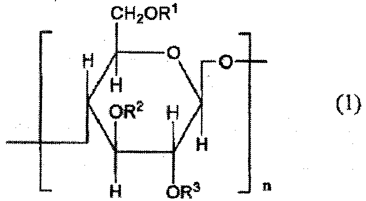 wobei R¹, R² und R³ gleich oder verschieden sind und ein Wasserstoffatom, eine niedere Alkylgruppe, eine -[CH₂CH₂₋ₖ(CH₃)ₖO]ₘH-Gruppe oder eine - CH₂CH(OH)CH₂OCⱼH₂ⱼ₊₁-Gruppe sind; n eine ganze Zahl von 100 bis 10000 ist; k eine ganze Zahl von 0 oder 1 ist; m eine ganze Zahl von 1 bis 10 ist; und j eine ganze Zahl von 6 bis 26 ist; und die hydrophobisch modifizierte Alkylcellulose notwendigerweise eine - CH₂CH(OH)CH₂OCⱼH₂ⱼ₊₁-Gruppe enthält, wobei die niedere Alkylgruppe eine Methylgruppe oder eine Ethylgruppe ist;
wobei das Verfahren Folgendes umfasst:
das Mischen, vor der Verwendung, eines Präparats A, das mindestens ein Salz enthält ausgewählt aus der Gruppe bestehend aus einem Carbonat und Bicarbonat, und eines Präparats B, das eine wasserlösliche Säure enthält, wobei das Präparat A und das Präparat B getrennt gehalten worden sind.

11. Verfahren zur Verwendung eines schaumartigen externen Hautpräparats, wobei das schaumartige externe Hautpräparat Folgendes umfasst: eine hydrophobisch modifizierte Alkylcellulose; mindestens ein Salz ausgewählt aus der Gruppe bestehend aus einem Carbonat und Bicarbonat; und eine wasserlösliche Säure, wobei die hydrophobisch modifizierte Alkylcellulose durch die folgende strukturelle Formel (1) dargestellt ist: 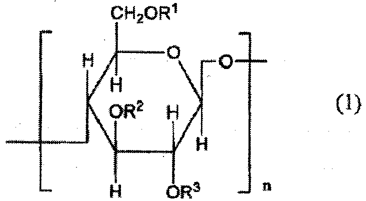 wobei R¹, R² und R³ gleich oder verschieden sind und ein Wasserstoffatom, eine niedere Alkylgruppe, eine -[CH₂CH₂₋ₖ(CH₃)ₖO]ₘH-Gruppe oder eine - CH₂CH(OH)CH₂OCⱼH₂ⱼ₊₁-Gruppe sind; n eine ganze Zahl von 100 bis 10000 ist; k eine ganze Zahl von 0 oder 1 ist; m eine ganze Zahl von 1 bis 10 ist; und j eine ganze Zahl von 6 bis 26 ist; und die hydrophobisch modifizierte Alkylcellulose notwendigerweise eine - CH₂CH(OH)CH₂OCⱼH₂ⱼ₊₁-Gruppe enthält, wobei die niedere Alkylgruppe eine Methylgruppe oder eine Ethylgruppe ist; und wobei das schaumartige externe Hautpräparat ein Präparat A, das mindestens ein Salz enthält ausgewählt aus der Gruppe bestehend aus einem Carbonat und Bicarbonat, und ein Präparat B umfasst, das eine wasserlösliche Säure enthält, wobei das Präparat A und das Präparat B getrennt gehalten und vor der Verwendung gemischt worden sind.

12. Schaumartiges externes Hautpräparat nach irgendeinem der Ansprüche 1-9, wobei das schaumartige externe Hautpräparat ein für das Gesicht verwendetes kosmetisches Packungspräparat ist.

## Revendications

1. Préparation cutanée externe de type mousse, comprenant: une alkyl cellulose modifiée hydrophobiquement; au moins un sel choisi dans le groupe constitué par un carbonate et un bicarbonate; et un acide hydrosoluble, dans laquelle l'alkyl cellulose modifiée hydrophobiquement est représentée par la formule structurale (1) suivante: 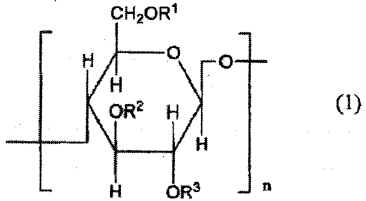 dans laquelle R¹, R² et R³ sont identiques ou différents, et sont un atome d'hydrogène, un groupe alkyle inférieur, un groupe -[CH₂CH₂₋ₖ(CH₃)ₖO]ₘH ou un groupe -CH₂CH(OH)CH₂OCⱼH₂ⱼ₊₁; n est un entier de 100 à 10000; k est un entier de 0 ou 1; m est un entier de 1 à 10; et j est un entier de 6 à 26; et l'alkyl cellulose modifiée hydrophobiquement contient nécessairement un groupe -CH₂CH(OH)CH₂OCⱼH₂ⱼ₊₁;
dans laquelle le groupe alkyle inférieur est un groupe méthyle ou un groupe éthyle.

2. Préparation cutanée externe de type mousse selon la revendication 1, dans laquelle l'alkyl cellulose modifiée hydrophobiquement représentée par la formule structurale (1) contient 10,0 à 50,0% en masse d'un groupe alkyle inférieur, 3,0 à 20,0% en masse d'un groupe -[CH₂CH₂₋ₖ(CH₃)ₖO]ₘH et 0,1 à 10,0% en masse d'un groupe-CH₂CH(OH)CH₂OCⱼH₂ⱼ₊₁.

3. Préparation cutanée externe de type mousse selon la revendication 1 ou 2, dans laquelle la valeur de j du groupe -CH₂CH(OH)CH₂OCⱼH₂ⱼ₊₁ dans la formule structurale (1) est 18.

4. Préparation cutanée externe de type mousse selon l'une quelconque des revendications 1 à 3, dans laquelle ledit au moins un sel choisi dans le groupe constitué par un carbonate et un bicarbonate est le bicarbonate de sodium.

5. Préparation cutanée externe de type mousse selon l'une quelconque des revendications 1 à 4, dans laquelle l'acide hydrosoluble est un hydroxy acide.

6. Préparation cutanée externe de type mousse selon la revendication 5, dans lequel l'hydroxy acide est l'acide citrique.

7. Préparation cutanée externe de type mousse selon l'une quelconque des revendications 1 à 6, comprenant en outre un polyalcool.

8. Préparation cutanée externe de type mousse selon la revendication 7, dans laquelle le polyalcool contient un ou plusieurs éléments choisis dans le groupe constitué par le propylène glycol, le dipropylène glycol, le 1,3-butylène glycol et le 1,2-pentanediol.

9. Préparation cutanée externe de type mousse selon l'une quelconque des revendications 1 à 8, laquelle préparation cutanée externe de type mousse comprend une préparation A contenant au moins un sel choisi dans le groupe constitué par un carbonate et un bicarbonate et une préparation B contenant un acide hydrosoluble, la préparation A et la préparation B ayant été maintenues séparées et mélangées avant l'utilisation.

10. Procédé pour préparer une préparation cutanée externe de type mousse, dans lequel la préparation cutanée externe de type mousse comprend: une alkyl cellulose modifiée hydrophobiquement; au moins un sel choisi dans le groupe constitué par un carbonate et un bicarbonate; et un acide hydrosoluble, dans lequel l'alkyl cellulose modifiée hydrophobiquement est représentée par la formule structurale (1) suivante: 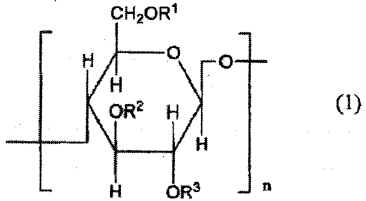 dans laquelle R¹, R² et R³ sont identiques ou différents, et sont un atome d'hydrogène, un groupe alkyle inférieur, un groupe -[CH₂CH₂₋ₖ(CH₃)ₖO]ₘH ou un groupe -CH₂CH(OH)CH₂OCⱼH₂ⱼ₊₁; n est un entier de 100 à 10000; k est un entier de 0 ou 1; m est un entier de 1 à 10; et j est un entier de 6 à 26; et l'alkyl cellulose modifiée hydrophobiquement contient nécessairement un groupe -CH₂CH(OH)CH₂OCⱼH₂ⱼ₊₁, dans laquelle le groupe alkyle inférieur est un groupe méthyle ou un groupe éthyle;
le procédé comprenant:
le mélange, avant l'utilisation, d'une préparation A contenant au moins un sel choisi dans le groupe constitué par un carbonate et un bicarbonate et d'une préparation B contenant un acide hydrosoluble, la préparation A et la préparation B ayant été maintenues séparées.

11. Procédé d'utilisation d'une préparation cutanée externe de type mousse, dans lequel la préparation externe de type mousse comprend: une alkyl cellulose modifiée hydrophobiquement; au moins un sel choisi dans le groupe constitué par un carbonate et un bicarbonate; et un acide hydrosoluble, dans lequel l'alkyl cellulose modifiée hydrophobiquement est représentée par la formule structurale (1) suivante: 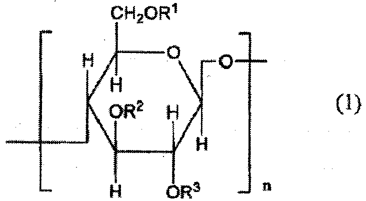 dans laquelle R¹, R² et R³ sont identiques ou différents, et sont un atome d'hydrogène, un groupe alkyle inférieur, un groupe -[CH₂CH₂₋ₖ(CH₃)ₖO]ₘH ou un groupe -CH₂CH(OH)CH₂OCⱼH₂ⱼ₊₁; n est un entier de 100 à 10000; k est un entier de 0 ou 1; m est un entier de 1 à 10; et j est un entier de 6 à 26; et l'alkyl cellulose modifiée hydrophobiquement contient nécessairement un groupe -CH₂CH(OH)CH₂OCⱼH₂ⱼ₊₁, dans laquelle le groupe alkyle inférieur est un groupe méthyle ou un groupe éthyle; et dans lequel la préparation cutanée externe de type mousse comprend une préparation A contenant au moins un sel choisi dans le groupe constitué par un carbonate et un bicarbonate et une préparation B contenant un acide hydrosoluble, la préparation A et la préparation B ayant été maintenues séparées et mélangées avant l'utilisation.

12. Préparation cutanée externe de type mousse selon l'une quelconque des revendications 1 à 9, laquelle préparation cutanée externe de type mousse est un matériau pour produit cosmétique utilisé pour le visage.
